# EUROPEAN PATENT SPECIFICATION

(11) **EP 0 558 657 B1**
(45) Date of publication and mention of the grant of the patent: **08.01.1997**
(21) Application number: 92901691.3
(22) Date of filing: 18.11.1991
(51) Int. Cl.: C07D 215/48, C07D 295/13, C07C 311/46, C07C 311/47, C07C 317/50, C07C 323/60, C07C 275/14, C07C 275/16, C07C 275/24, C07C 275/26, C07K 5/02

(54) **RETROVIRAL PROTEASE INHIBITORS**
RETROVIRALE PROTEASE INHIBITOREN
INHIBITEURS DE PROTEASES RETROVIRALES

(30) Priority: 19.11.1990 US 615210; 14.11.1991 US 789643
(43) Date of publication of application: 08.09.1993
(60) Divisional application: 96107357.4
(73) Proprietor: MONSANTO COMPANY, St. Louis Missouri 63167 (US)
(72) Inventor: REED, Kathryn, Lea, Richmond Heights, MO 63117 (US); TALLEY, John, Jeffrey, Chesterfield, MO 63017 (US)
(74) Representative: Ernst, Hubert
(86) International application number: US9108596
(87) International publication number: WO9208700

(56) References cited:
- EP-A- 0 172 347
- EP-A- 0 223 437
- EP-A- 0 264 795
- EP-A- 0 389 898
- Journal of Medicinal Chemistry, vol. 30, no. 7, July 1987, (Washington, US), S. ROSENBERG et al.: "Novel renin inhibitors containing analogues of statine retro-inverted at the C-termini: Specificity at the P2 histidine site", page 1224-1228, see page 1225, table 1

## Description

### BACKGROUND OF THE INVENTION

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8Å Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions.

### DETAILED DESCRIPTION OF THE INVENTION

In accordance with the present invention, there is provided a retroviral protease inhibiting compound of the formula: 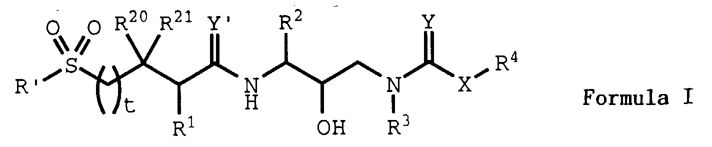 or a pharmaceutically acceptable salt thereof, wherein t represents 0 or 1;
R' represents alkyl, aryl or aralkyl radicals;
R¹ represents -CH₂SO₂NH₂, alkyl of 1-4 carbon atoms or cycloalkyl radicals or the side chain of the amino acid asparagine, S-methyl cysteine or the sulfoxide or sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid or beta-cyano alanine;
R² represents alkyl, aryl, cycloalkyl, cycloalkylalkyl or aralkyl radicals optionally substituted with halogen, -OR⁹ or SR⁹ radicals, wherein R⁹ represents hydrogen or alkyl radicals;
R³ and R⁴ independently represent alkyl, cycloalkyl, cycloalkylalkyl, heterocycloalkyl, heterocycloalkylalkyl, aryl, aralkyl or heteroaralkyl radicals;
R²⁰ and R²¹ independently represent hydrogen or alkyl radicals;
X represents O and CH(R¹⁷), wherein R¹⁷ represents hydrogen or alkyl radicals; and
Y and Y' independently represent O or S; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido.

As utilized herein, the term "alkyl", alone or in combination, means a straight-chain or branched-chain alkyl radical containing from 1 to 8 carbon atoms. Examples of such radicals include methyl, ethyl, n-propyl, isopropyl, n-butyl, isobutyl, sec-butyl, tert-butyl, pentyl, isoamyl, hexyl, octyl. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy. The term "cycloalkyl" means an alkyl radical which contains from 3 to 8 carbon atoms and is cyclic. The term "cycloalkylalkyl" means an alkyl radical as defined above which is substituted by a cycloalkyl radical containing from 3 to 8, preferably from 3 to 6, carbon atoms. Examples of such cycloalkyl radicals include cyclopropyl, cyclobutyl, cyclopentyl, cyclohexyl. The term "aryl", alone or in combination, means a phenyl or naphthyl radical which optionally carries one or more substituents selected from alkyl, alkoxy, halogen, hydroxy, amino and the like, such as phenyl, p-tolyl, 4-methoxyphenyl, 4-(tert-butoxy)phenyl, 4-fluorophenyl, 4-chlorophenyl, 4-hydroxyphenyl, 1-naphthyl, 2-naphthyl. The term "aralkyl", alone or in combination, means an alkyl radical as defined above in which one hydrogen atom is replaced by an aryl radical as defined above, such as benzyl, 2-phenylethyl. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl, 4-methylvaleryl. The term "cycloalkylcarbonyl" means an acyl group derived from a monocyclic or bridged cycloalkanecarboxylic acid such as cyclopropanecarbonyl, cyclohexanecarbonyl, adamantanecarbonyl, or from a benz-fused monocyclic cycloalkanecarboxylic acid which is optionally substituted by, for example, alkanoylamino, such as 1,2,3,4-tetrahydro-2-naphthoyl,2-acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl,4-methoxyhydrocinnamoyl. The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl, 3-(benzyloxyformamido)-2-naphthoyl. The heterocyclyl or heterocycloalkyl portion of a heterocyclylcarbonyl, heterocyclyloxycarbonyl, heterocyclylalkoxycarbonyl, or heterocyclyalkyl group or the like is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy, oxo, and the like, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl), pyrazolyl, pyridyl, pyrazinyl, pyrimidinyl, furyl, thienyl, triazolyl, oxazolyl, thiazolyl, indolyl (e.g., 2-indolyl), quinolinyl, (e.g., 2-quinolinyl, 3-quinolinyl, 1-oxido-2-quinolinyl), isoquinolinyl (e.g., 1-isoquinolinyl, 3-isoquinolinyl), tetrahydroquinolinyl (e.g., 1,2,3,4-tetrahydro-2-quinolyl), 1,2,3,4-tetrahydroisoquinolinyl (e.g., 1,2,3,4-tetrahydro-1-oxo-isoquinolinyl), quinoxalinyl, β-carbolinyl, benzofurancarbonyl, benzimidazolyl radicals. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl, cycloalkylalkyl radicals. The term "halogen" means fluorine, chlorine, bromine or iodine. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR. Preferred leaving groups are indicated herein where appropriate.

Procedures for preparing the compounds of Formula I are set forth below. It should be noted that the general procedure is shown as it relates to preparation of compounds having the specified stereochemistry, for example, wherein the stereochemistry about the hydroxyl group is designated as (R). However, such procedures are generally applicable, as illustrated, to those compounds of opposite configuration, e.g., where the stereochemistry about the hydroxyl group is (S).

### Preparation of Compounds of Formula I

The compounds of the present invention represented by Formula I above can be prepared utilizing the following general procedure. An N-protected chloroketone derivative of an amino acid having the formula: 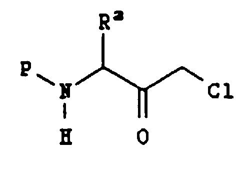 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to about 25°C, preferably at about 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 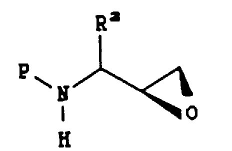 wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from about 10°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formula: 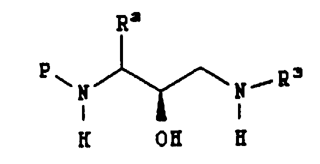 wherein P, R² and R³ are as described above.

Where X is either O or C, the appropriate analogs can be prepared by reacting the above described amino alcohol with an acid chloride or anhydride to form the analog wherein X is C, or with a chloroformate or pyrocarbonate where X is O. Procedures for reacting these compounds with an amine are well known in the art. Examples of such compounds include t-butylacetyl chloride, acetic anhydride, t-butyl pyrocarbonate, and butyl chloroformate. These analogs can be represented by the formulas:

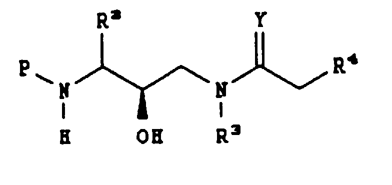

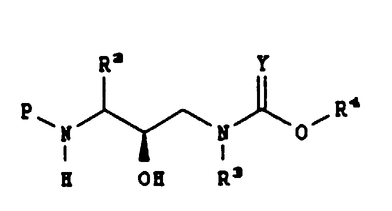

The derivative of the amino alcohol and the corresponding sulfur analog can be represented by the formula: 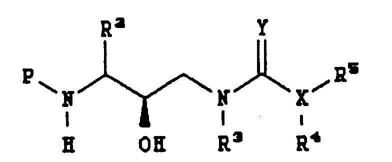 R⁵ is absent when X is O and R⁵ is hydrogen when X is C(R¹⁷).

Following preparation of such derivatives, the amino protecting group P is removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then reacted with the free acid of the formula II

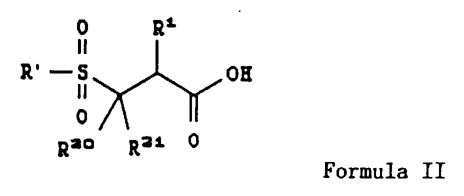

### Preparation of Compounds of Formula II

A mercaptan of the formula R'SH is reacted with a substituted methacrylate of the formula: 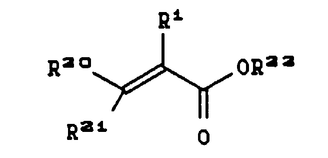 by way of a Michael Addition. The Michael Addition is conducted in a suitable solvent and in the presence of a suitable base, to produce the corresponding thiol derivative represented by the formula: 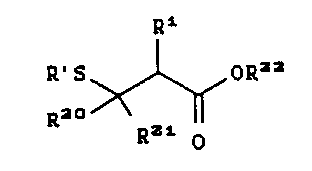 wherein R' and R¹ represent radicals defined above; R²⁰ and R²¹ represent hydrogen and radicals as defined for R¹; and R²² represents radicals as defined by R³. Suitable solvents in which the Michael Addition can be conducted include alcohols such as, for example, methanol, ethanol, butanol and the like, as well as ethers, e.g., THF, and acetonitrile, DMF, DMSO, and the like, including mixtures thereof. Suitable bases include Group I metal alkoxides such as, for example sodium methoxide, sodium ethoxide, sodium butoxide and the like as well as Group I metal hydrides, such as sodium hydride, including mixtures thereof.

The thiol derivative is converted into the corresponding sulfone of the formula: 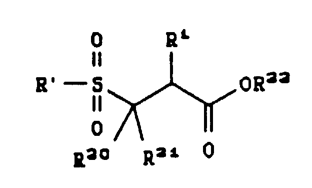 by oxidizing the thiol derivative with a suitable oxidation agent in a suitable solvent. Suitable oxidation agents include, for example, hydrogen peroxide, sodium meta-perborate, oxone (potassium peroxy monosulfate), meta-chloroperoxybenzoic acid, and the like, including mixtures thereof. Suitable solvents include acetic acid (for sodium meta-perborate) and, for other peracids, ethers such as THF and dioxane, and acetonitrile, DMF and the like, including mixtures thereof.

The sulfone is then converted to the corresponding free acid of the formula: 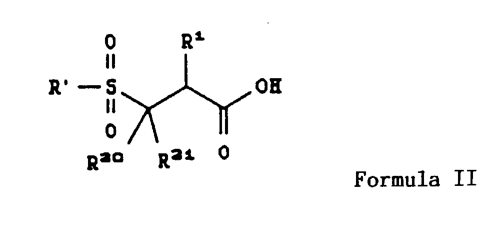 utilizing a suitable base, e.g., lithium hydroxide, sodium hydroxide and the like, including mixtures thereof, in a suitable solvent, such as, for example, THF, acetonitrile, DMF, DMSO, methylene chloride and the like, including mixtures thereof..

The free acid is then coupled, utilizing, as described above, procedures well known in the art, to the urea derivative, or analog thereof.

Alternatively, one can couple the urea isostere to the commercially available acid, 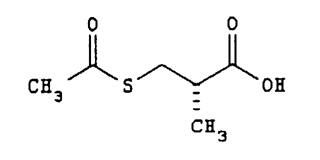 remove the thioacetyl group with a suitable base, such as hydroxide, or an amine, such as ammonia, and then react the resulting thiol with an alkylating agent, such as an alkyl halide, tosylate or mesylate to afford compounds at the following structure:

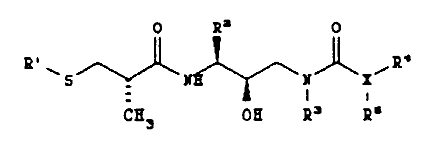

The sulfur can then be oxidized to the corresponding sulfone using suitable oxidizing agents, as described above, to afford the desired compounds of the following structure: 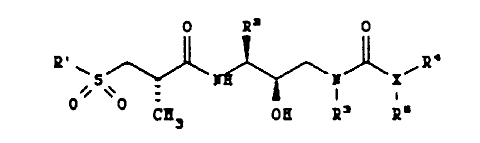 Alternatively, to prepare compounds of Formula I, a substituted methacrylate of the formula: 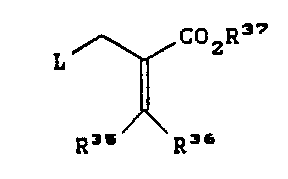 wherein L represents a leaving group as previously defined, R³⁵ and R³⁶ represent hydrogen and radicals as defined for R¹; and R³⁷ represents alkyl, aralkyl, cycloalkyl and cycloalkylalkyl radicals,
is reacted with a suitable sulfonating agent, such as, for example, a sulfinic acid represented by the formula R'SO₂M, wherein R' represents radicals as defined above and M represents a metal adapted to form a salt of the acid, e.g., sodium, to produce the corresponding sulfone represented by the formula: 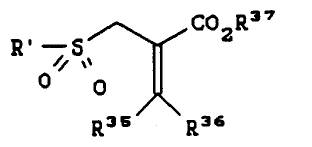 wherein R', R³⁵, R³⁶ and R³⁷ are as defined above. The sulfone is then hydrolyzed in the presence of a suitable base, such as lithium hydroxide, sodium hydroxide and the like, to the compound represented by the formula: 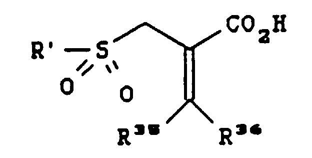 wherein R', R³⁵ and R³⁶ represent radicals as defined above. The resulting compound is then asymmetrically hydrogenated utilizing an asymmetric hydrogenation catalyst such as, for example, a ruthenium-BINAP complex, to produce the reduced product, substantially enriched in the more active isomer, represented by the formula: 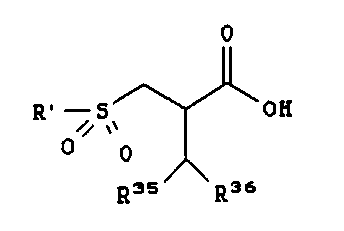 wherein R', R³⁵ and R³⁶ represent radicals as defined above. Where the more active isomer has the R-stereochemistry, a Ru(R-BINAP) asymmetric hydrogenation catalyst can be utilized. Conversely, where the more active isomer has the S-sterochemistry, a Ru(S-BINAP) catalyst can be utilized. Where both isomers are active, or where it is desired to have a mixture of the two diastereomers, a hydrogenation catalyst such as platinum, or palladium, on carbon can be utilized to reduce the above compound. The reduced compound is then coupled to the amino alcohol derivatives, as described above, to produce compounds of Formula I

It is contemplated that where R³ of the amino alcohol intermediate is hydrogen, the inhibitor compounds scan be prepared through reductive amination of the final product of the reaction between the amino alcohol and the amine or at any other stage of the synthesis for preparing the inhibitor compounds.

Contemplated equivalents of the general formula set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Examples 1-4 illustrate compounds wherein X is N rather than O or C(R¹⁷). However, the nitrogen can be replaced by replacing the isocyanate R⁴NCO with an acid chloride or anhydride where X is C, or with a chloroformate or pyrocarbonate where X is O, to produce the compounds of the present invention.

All reagents were used as received without purification. All proton and carbon NMR spectra were obtained on either a Varian VXR-300 or VXR-400 nuclear magnetic resonance spectrometer.

### Example 1A

The procedure described below illustrates preparation of compounds of Formula I
Propanamide, N-[3-[[[(1,1-dimethylethyl)amino]carbonyl](3-methylbutyl)amino]-2-hydroxy-1-(phenylmethyl)propyl]-2-methyl-3-(2-phenylethylsulfonyl)-,[1S-[1R*(R*),2S*]] and its diastereomer.

### Part A

A solution of methyl methacrylate (7.25 g, 72.5 mmol) and phenethyl mercaptan (10.0 g, 72.5 mmol) in 100 mL of methanol was cooled in an ice bath and treated with sodium methoxide (100 mg, 1.85 mmol). The solution was stirred under nitrogen for 3 h and then concentrated *in vacuo* to give an oil that was taken up in ether and washed with 1 N aqueous potassium hydrogen sulfate, saturated aqueous sodium chloride, dried over anhydrous magnesium sulfate, filtered and concentrated to give 16.83 g, 97.5% of methyl 2-(R,S)-methyl-4-thia-6-phenyl hexanoate as an oil. TLC on SiO₂ eluting with 20:1 hexane:ethyl acetate (v:v) R_{f}=0.41.

### Part B

A solution of methyl 2-(R,S)-methyl-4-thia-6-phenyl hexanoate (4.00 g, 16.8 mmol) in 100 mL of dichloromethane was stirred at room temperature and treated portion wise with meta-chloroperoxybenzoic acid (7.38 g, 39.2 mmol) over approximately 40 m. The solution was stirred at room temperature for 16 h and then filtered and the filterate washed with saturated aqueous sodium bicarbonate, 1N sodium hydroxide, saturated aqueous sodium chloride, dried over anhydrous magnesium sulfate, filtered, and concentrated to give 4.50 g, 99% of desired sulfone. The unpurified sulfone was dissolved in 100 mL of tetrahydrofuran and treated with a solution of lithium hydroxide (1.04 g, 24.5 mmol) in 40 mL of water. The solution was stirred at room temperature for 2 m and then concentrated *in vacuo.* The residue was then acidified with 1N aqueous potassium hydrogen sulfate to pH=1 and then extracted three times with ethyl acetate. The combined ethyl acetate solution was washed with saturated aqueous sodium chloride, dried over anhydrous magnesium sulfate, filtered and concentrated to give a white solid. The solid was taken up in boiling ethyl acetate/hexane and allowed to stand undisturbed whereupon white needles formed that were isolated by filtration and air dried to give 3.38 g, 79% of 2-(R,S)-methyl-3(β-phenethylsulfonyl)-propionic acid, mp 91-93°C.

### Part C

A solution of 2-(R,S)-methyl-3(β-phenethylsulfonyl)-propionic acid (166.1 mg, 0.65 mmol), N-hydroxybenzotriazole (HOBT) (146.9 mg, 0.97 mmol), and 1-(3-dimethylaminopropyl)-3-ethylcarbodiimide hydrochloride (EDC) (145.8 mg, 0.75 mmol) in 4 mL of anhydrous dimethylformamide (DMF) cooled to 0°C and stirred under nitrogen for 0.5 h. This solution was then treated with 3-[[(dimethylethyl)amino]carbonyl](3-methylbutyl)amino-2(R)-hydroxy-1(S)-(phenylmethyl)propyl amine (201.9 mg, 0.59 mmol) and stirred at room temperature for 16 h. The solution was poured into 30 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 110.0 mg, 32% of (2R,3S)-3-[N-2-(R)-methyl-3-(β-phenethylsulfonyl)propionyl]amido-1-isoamyl-1-(tert-butylcarbamoyl)amino-4-phenyl-2-butanol and (2R,3S)-3-[N-2-(S)-methyl-3-(β-phenethylsulfonyl)propionyl]amido-1-isoamyl-1-(tert-butylcarbamoyl)amino-4-phenyl-2-butanol, FAB mass spectrum (MH+) =588. Flash chromatography of the mixture on silica gel eluting with 1:1 hexane:ethyl acetate afforded the separated diastereomers.

### Example 1B

Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)- [1S-[1R*(R*), 2S*]], and its diastereomer.

### Part A

A solution of methyl 2-(bromomethyl)-acrylate (26.4 g, 0.148 mol) in 100 mL of methanol was treated with sodium methanesulfinate (15.1 g, 0.148 mol) portion wise over 10 m at room temperature. The solution was then stirred at room temperature for a period of 1.25 h and the solution concentrated *in vacuo.* The residue was then taken up in water and extracted four times with ethyl acetate. The combined ethyl acetate solution was washed with saturated sodium chloride, dried over anhydrous magnesium sulfate, filtered and concentrated to give a white solid, 20.7 g which was taken up in boiling acetone/methyl tert-butyl ether and allowed to stand whereupon crystals of pure methyl 2-(methylsulfonylmethyl) acrylate 18.0 g, 68% formed, mp 65-68 0°C.

### Part B

A solution of methyl 2-(methylsulfonylmethyl) acrylate (970 mg, 5.44 mmol) in 15 mL of tetrahydrofuran was treated with a solution of lithium hydroxide (270 mg, 6.4 mmol) in 7 mL of water. The solution was stirred at room temperature for 5 m and then acidified to pH=1 with 1 N aqueous potassium hydrogen sulfate and the solution extracted three times with ethyl acetate. The combined ethyl acetate solution was dried over anhydrous magnesium sulfate, filtered, and concentrated to give 793 mg, 89% of 2-(methylsulfonylmethyl) acrylic acid, mp 147-149 0°C.

### Part C

A solution of 2-(methylsulfonylmethyl) acrylic acid (700 mg, 4.26 mmol) in 20 mL of methanol was charged into a Fisher-Porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtered through a pad of celite to remove the catalyst and the filterate concentrated *in vacuo* to give 682 mg 96% of 2-(R,S)-methyl-3-methylsulfonyl propionic acid.

### Part D

A solution of 2-(R,S)-methyl-3(methylsulfonyl) propionic acid (263.5 mg, 1.585 mmol), N-hydroxybenzotriazole (HOBT) (322.2 mg, 2.13 mmol), and 1-(3-dimethylaminopropyl)-3-ethylcarbodiimide hydrochloride (EDC) (339.1 mg, 1.74 mmol) in 4 mL of anhydrous dimethylformamide (DMF) cooled to 0°C and stirred under nitrogen for 0.5 h. This solution was then treated with 3-[[(1,1-dimethylethyl)amino]carbonyl](3-methylbutyl)amino-2(R)-hydroxy-I(S)-(phenylmethyl)propyl amine (543.5 mg, 1.58 mmol) and stirred at room temperature for 16 h. The solution was poured into 60 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 471.8 mg, 60% of Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*), 2S*]]- and its diastereomer.

### Example 2

### Preparation of Sulfone Inhibitors From L-(+)-S-acetyl-β-mercaptoisobutyric Acid

### Part A:

Propanamide, N-[3-[[[(1,1-dimethylethyl)amino]carbonyl](3-methylbutyl)amino]-2-hydroxy-1-(phenylmethyl)propyl]-2-methyl-3-S-acetyl)-[1S-[1R*),2S*]]-.

A round-bottomed flask was charged with (2R,3R)-3-amino-1-isoamyl-1-(tert-butylcarbamoyl)amino-4-phenyl-2-butanol (901.5 mg, 2.575 mmol), L-(+)-S-acetyl-b-mercaptoisobutyric acid (164.5 mg, 2.575 mmol), 1-(3-dimethylaminopropyl)-3-ethylcarbodiimide hydrochloride (EDC) (339.1 mg, 1.74 mmol), and 10 mL of CH₂Cl₂ and allowed to stir at room temperature for 16 h. The solution was concentrated in vacuo and the residue taken up in ethyl acetate, washed with 1N KHSO₄ sat. aq. NaHCO₃, brine, dried over anhydrous MgSO₄, filtered and concentrated to give an oil that was purified by radial chromatography on SiO₂ eluting with ethyl acetate to give the pure product, 800 mg, 63%.

### Part B:

Propanamide, N-[3-[[[1,1-dimethylethyl)amino]carbonyl](3-methylbutyl)amino]-2-hydroxy-1-(phenylmethyl)propyl]-2-methyl-3-mercapto)-, [1S-[1R*(R*),2S*]]-.

A solution of [1S-[1R*(R*),2S*]]- N-[3-[[[(1,1-dimethylethyl)amino]carbonyl](3-methylbutyl)amino]-2-hydroxy-1-(phenylmethyl)propyl]-2-methyl-3-S-acetyl)-propanamide (420 mg, 0.85 mmol) in 10 mL of methanol was treated with anhydrous ammonia for ca. 1 m at 0°C. The solution was stirred at that temperature for 16 h and then concentrated in vacuo to give 380 mg, 99%, of the desired product that was used directly in the next step without further purification.

### Part C:

Propanamide, N-[3-[[[(1,1-dimethylethyl)amino]carbonyl](3-methylbutyl)amino]-2-hydroxy-1-(phenylmethyl)propyl]-2-methyl-3-S-methyl-, [1S-[1R*(R*),2S*]]-.

A solution of [1S-[1R*(R*),2S*]]- N-[3-[[[(1,1-dimethylethyl)amino]carbonyl](3-methylbutyl)amino]-2-hydroxy-1-(phenylmethyl)propyl]-2-methyl-3-mercapto)-propanamide (380 mg, 0.841 mmol) in 10 mL of dry toluene under nitrogen was treated in rapid succession with 1,8-diazabicyclo[5.4.0]undec-7-ene, (DBU), (128.1 mg. 0.841 mmol) and iodomethane (119.0 mg, 0.841 mmol). After 0.5 h at room temperature the reaction was found to be complete and the solution was diluted with ethyl acetate washed with 1N KHSO₄, sat. aq. NaHCO₃, brine. After the solution was dried over anhydrous MgSO₄, filtered and concentrated in vacuo the desired product was obtained as white foam was obtained, 370 mg, 94.5%, that was used directed in the next step.

### Part D:

Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*),2S*]]-.

A solution of [1S-[1R*(R*),2S*]]-N-[3-[[[(1,1-dimethylethyl)amino]carbonyl](3-methylbutyl)amino]-2-hydroxy-1-(phenylmethyl)propyl]-2-methyl-3-S-methyl)-propanamide (340 mg, 0.73 mmol) and sodium perborate (500 mg, 3.25 mmol) in 30 mL of glacial acetic acid was warmed to 55°C for 16 h. The solution was concentrated in vacuo and then the residue taken up in ethyl acetate, washed with water, sat. aq. NaHCO₃, brine, dried over anhydrous MgSO₄, filtered and concentrated to give the desired product as a white solid, 350 mg, 96%.

### Example 3

The compounds shown in Table 1 were prepared generally according to the procedure set forth in Examples 1 and 2.

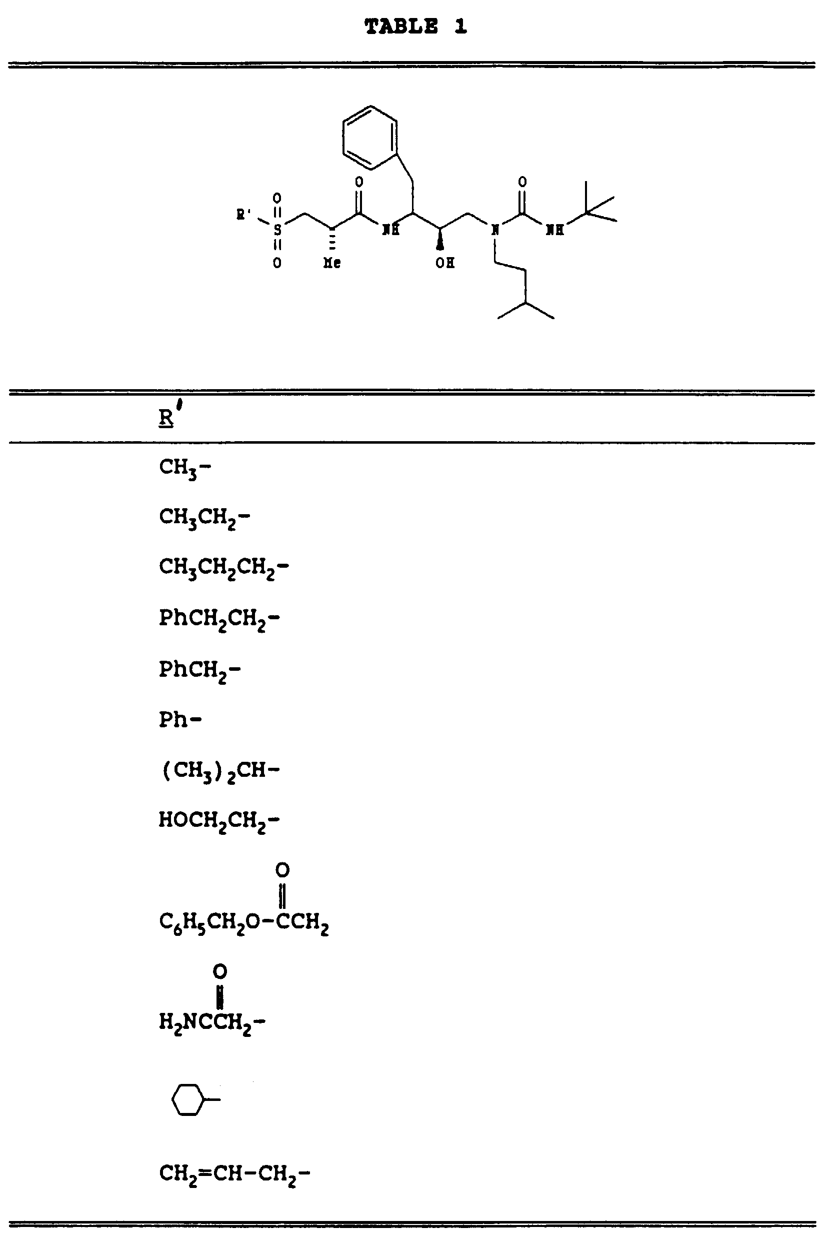

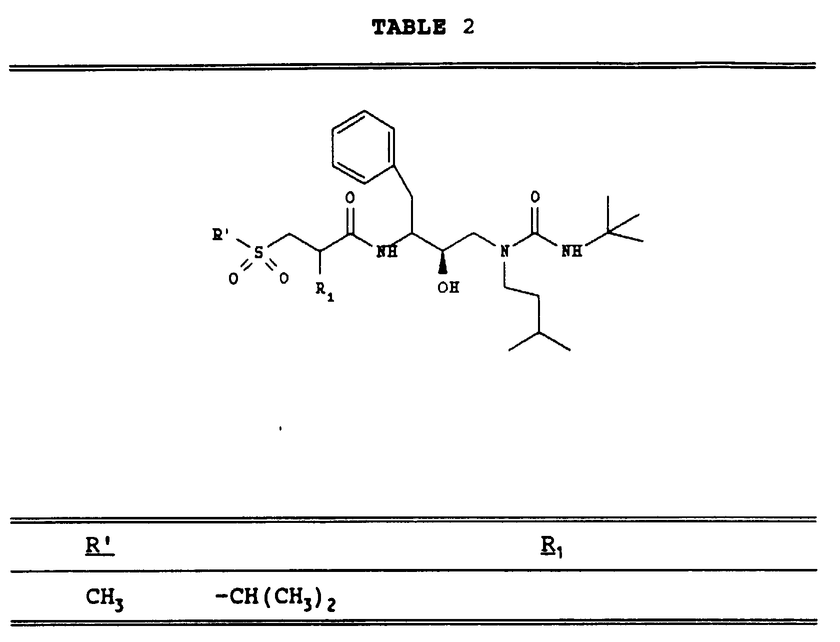

### Example 4

### Preparation of 2(S)-methyl-3-(methylsulfonyl)propionic Acid.

To a solution of 10g of D-(-)-S-benzoyl-b-mercaptioisobutyric acid t-butyl ester in 20 mL of methanol was bubbled in gaseous ammonia at 0°C. The reaction was allowed to then warm to room temperature, stirred overnight and concentrated under reduced pressure. The resulting mixture of a solid (benzamide) and liquid was filtered to provide 5.21g of a pale oil which then solidified. This was identified as 2(S)-methyl-3-mercaptopropionic aid t-butyl ester.

To a solution of 5.21g of 2(S)-methyl-3-mercaptopropionic acid t-butyl ester in 75 mL of toluene at 0°C was added 4.50g of 1,8-diazabicyclo[5.40]undec-7-ene and 1.94 mL of methyl iodide. After stirring at room temperature for 2.5 hours, the volatiles were removed, ethyl acetate added, washed with dilute hydrochloric acid, water, brine, dried and concentrated to afford 2.82g of a pale oil, identified as 2(S)-methyl-3-(thiomethyl)propionic acid t-butyl ester.

To a solution of 2.82g of 2(S)-methyl-3-(thiomethyl)propionic acid t-butyl ester in 50 mL of acetic acid was added 5.58g of sodium perborate and the mixture heated to 55°C for 17 hours. The reaction was poured into water, extracted with methylene chloride, washed with aqueous sodium bicarbonate, dried and concentrated to afford 2.68g of 2(S)-methyl-3-(methylsulfonyl)propionic acid t-butyl ester as a white solid.

To 2.68g of 2(S)-methyl-3-(methylsulfonyl)propionic acid t-butyl ester was added 20 mL of 4N hydrochlorid acid/dioxane and the mixture stirred at room temperature for 19 hours. The solvent was removed under reduced pressure to afford 2.18g of crude product, which was recrystallized from ethyl acetate/hexane to yield 1.44g of 2(S)-methyl-3-(methylsulfonyl)propionic acid as white crystals.

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncitial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

## Claims

1. Compound represented by the formula: 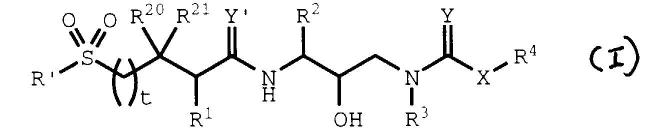 or a pharmaceutically acceptable salt thereof, wherein t represents 0 or 1;
R' represents alkyl, aryl or aralkyl radicals;
R¹ represents -CH₂SO₂NH₂, alkyl of 1-4 carbon atoms or cycloalkyl radicals or the side chain of the amino acid asparagine, S-methyl cysteine or the sulfoxide or sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid or beta-cyano alanine;
R² represents alkyl, aryl, cycloalkyl, cycloalkylalkyl or aralkyl radicals optionally substituted with halogen, -OR⁹ or SR⁹ radicals, wherein R⁹ represents hydrogen or alkyl radicals;
R³ and R⁴ independently represent alkyl, cycloalkyl, cycloalkylalkyl, heterocycloalkyl, heterocycloalkylalkyl, aryl, aralkyl or heteroaralkyl radicals;
R²⁰ and R²¹ independently represent hydrogen or alkyl radicals;
X represents O and CH(R¹⁷), wherein R¹⁷ represents hydrogen or alkyl radicals; and
Y and Y' independently represent O or S; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with a C₁-C₈ alkyl, C₁-C₈ alkoxy, halogen, hydroxy or amino radical; heterocycloalkyl, alone or in combination, is pyrrolidinyl, piperidinyl, piperazinyl, morpholinyl, thiamorpholinyl, tetrahydroquinolinyl or tetrahydroisoquinolinyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido; and heteroaryl, alone or in combination, is pyrrolyl, imidazolyl, pyrazolyl, pyridyl, pyrazinyl, pyrimidinyl, furyl, thienyl, triazolyl, oxazolyl, thiazolyl, indolyl, quinolinyl, isoquinolinyl, quinoxalinyl, β-carbolinyl, benzofuranyl or benzimidazolyl radicals, which radicals are optionally substituted on a substitutable carbon atom with halogen, C₁-C₈ alkyl, C₁-C₈ alkoxy or oxo, on a ring secondary nitrogen atom with C₁-C₈ alkyl, aryl-C₁-C₈-alkoxycarbonyl, C₁-C₈ alkanoyl, phenyl or phenyl-C₁-C₈-alkyl, or on a ring tertiary nitrogen atom by oxido.

2. Compound of Claim 1 wherein t is 0;
R¹ represents hydrogen radical or alkyl radical of 1-4 carbon atoms; and
R² represents alkyl, cycloalkylalkyl or aralkyl radicals optionally substituted with halogen, -OR⁹ or SR⁹ radicals, wherein R⁹ represents alkyl radicals.

3. Compound of Claim 2 wherein R' represents methyl or phenethyl radicals;
R¹ represents methyl, ethyl, isopropyl or tert-butyl radicals;
R² represents CH₃SCH₂CH₂-, iso-butyl, n-butyl, benzyl, 4-fluorobenzyl, 2-naphthylmethyl or cyclohexylmethyl radicals;
R³ represents i-propyl, i-butyl, neo-pentyl, i-amyl, t-butyl, n-butyl, cyclohexylmethyl, benzyl, para-fluorobenzyl, para-methoxybenzyl, para-methylbenzyl, and 2-naphthylmethyl radicals;
R⁴ represents t-butyl radical; and
R²⁰ and R²¹ independently represent hydrogen or methyl radicals.

4. Compound of Claim 2 wherein R¹ represents hydrogen radical or alkyl radical of 1-4 carbon atoms;
R² represents alkyl, cycloalkylalkyl or aralkyl radicals;
R³ represents alkyl of 2 to 5 carbon atoms or heteroaralkyl radical; and
R⁴ represents tert-butyl radical.

5. A pharmaceutical composition comprising a compound according to any of claims 1 to 4 and a pharmaceutically acceptable carrier.

6. Use of a compound according to any of claims 1 to 4 for the manufacture of a medicament for virus inhibiting.

7. Use according to claim 6 of a compound defined in any of claims 1 to 4 for the manufacture of a medicament for inhibiting retroviral protease.

8. Use according to claim 7 wherein the retroviral protease is HIV protease.

9. Use according to claim 6 of a compound defined in any of claims 1 to 4 for the manufacture of a medicament for treatment of a retroviral infection.

10. Use according to claim 9 wherein the retroviral infection is an HIV infection.

11. Use according to claim 6 of a compound defined in any of claims 1 to 4 for the manufacture of a medicament for the treatment of AIDS.

## Patentansprüche

1. Verbindung, dargestellt durch die Formel 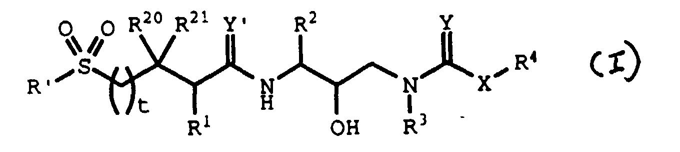 oder ein pharmazeutisch akzeptables Salz davon, worin t für 0 oder 1 steht;
R' für einen Alkyl-, Aryl- oder Aralkyl-Rest steht;
R¹ -CH₂SO₂NH₂, einen Alkyl-Rest mit 1-4 Kohlenstoffatomen oder einen Cycloalkyl-Rest oder die Seitenkette der Aminosäure Asparagin, S-Methyl-Cystein oder die Sulfoxid- oder Sulfon-Derivate davon, Glycin, Allo-Isoleucin, Alanin, Leucin, tert.-Leucin, Phenylalanin, Ornithin, Threonin, Allo-Threonin, Isoleucin, Histidin, Norleucin, Valin, Glutamin, Serin, Asparaginsäure oder Beta-Cyanoalanin bedeutet;
R² einen Alkyl-, Aryl-, Cycloalkyl-, Cycloalkylalkyl- oder Aralkyl-Rest, gegebenenfalls substituiert durch Halogen, einen -OR⁹ oder SR⁹-Rest bedeutet, worin R⁹ für einen Wasserstoff- oder Alkyl-Rest steht;
R³ und R⁴ unabhängig voneinander für einen Alkyl-, Cycloalkyl-, Cycloalkylalkyl-, Heterocycloalkyl-, Heterocycloalkylalkyl-, Aryl-, Aralkyl- oder Heteroaralkyl-Rest stehen;
R²⁰ und R²¹ unabhängig voneinander einen Wasserstoff- oder Alkyl-Rest bedeuten;
X für O und CH(R¹⁷) steht, worin R¹⁷ einen Wasserstoff- oder Alkyl-Rest bedeutet; und
Y und Y' unabhängig voneinander O oder S bedeuten; und
worin Alkyl alleine oder in Kombination ein geradkettiger oder verzweigtkettiger Alkyl-Rest ist, der 1 bis 8 Kohlenstoffatome enthält, Cycloalkyl alleine oder in Kombination ein cyclischer Alkyl-Rest mit 3 bis 8 Kohlenstoffatomen ist; Aryl alleine oder in Kombination ein unsubstituierter Phenyl- oder Naphthyl-Rest, oder ein Phenyl- oder Naphthyl-Rest ist, der durch einen C₁-C₈-Alkyl-, C₁-C₈-Alkoxy-, Halogen-, Hydroxy- oder Amino-Rest substituiert ist; Heterocycloalkyl alleine oder in Kombination ein Pyrrolidinyl-, Piperidinyl-, Piperazinyl-, Morpholinyl-, Thiamorpholinyl-, Tetrahydrochinolinyl- oder Tetrahydroisochinolinyl-Rest ist, welche Reste gegebenenfalls an einem substituierbaren Kohlenstoffatom durch Halogen, C₁-C₈-Alkyl, C₁-C₈-Alkoxy oder Oxo, an einem sekundären Stickstoffring-Atom durch C₁-C₈-Alkyl, Aryl-C₁-C₈-Alkoxycarbonyl, C₁-C₈-Alkanoyl, Phenyl oder Phenyl-C₁-C₈-Alkyl, oder an einem tertiären Stickstoffring-Atom durch Oxido substituiert sind; und Heteroaryl alleine oder in Kombination ein Pyrrolyl-, Imidazolyl-, Pyrazolyl-, Pyridyl-, Pyrazinyl-, Pyrimidinyl-, Furyl-, Thienyl-, Triazolyl-, Oxazolyl-, Thiazolyl-, Indolyl-, Chinolinyl-, Isochinolinyl-, Chinoxalinyl-, β-Carbolinyl-, Benzofuranyl- oder Benzimidazolyl-Rest ist, welche Reste gegebenenfalls an einem substituierbaren Kohlenstoffatom durch Halogen, C₁-C₈-Alkyl, C₁-C₈-Alkoxy oder Oxo, an einem sekundären Stickstoffring-Atom durch C₁-C₈-Alkyl, Aryl-C₁-C₈-Alkoxycarbonyl, C₁-C₈-Alkanoyl, Phenyl oder Phenyl-C₁-C₈-Alkyl, oder an einem tertiären Stickstoffring-Atom durch Oxido substituiert sind.

2. Verbindung nach Anspruch 1, worin t 0 ist;
R¹ einen Wasserstoff-Rest oder einen Alkyl-Rest mit 1-4 Kohlenstoffatomen bedeutet; und
R² einen Alkyl-, Cycloalkylalkyl- oder Aralkyl-Rest, gegebenenfalls substituiert durch einen Halogen-, -OR⁹- oder SR⁹-Rest darstellt, worin R⁹ einen Alkyl-Rest bedeutet.

3. Verbindung nach Anspruch 2, worin R' einen Methyl- oder Phenetyhl-Rest bedeutet;
R¹ einen Methyl-, Ethyl-, Isopropyl- oder tert.Butyl-Rest darstellt;
R² einen CH₃SCH₂CH₂-, Isobutyl-, n-Butyl-, Benzyl- 4-Fluorbenzyl-, 2-Naphthylmethyl- oder Cyclohexylmethyl-Rest bedeutet,
R³ einen i-Propyl-, i-Butyl-, neo-Pentyl-, i-Amyl-, t-Butyl-, n-Butyl-, Cyclohexylmethyl-, Benzyl-, para-Fluorbenzyl-, para-Methoxybenzyl-, para-Methylbenzyl- und 2-Naphthylmethyl-Rest bedeutet;
R⁴ einen t-Butyl-Rest darstellt; und
R²⁰ und R²¹ unabhängig voneinander einen Wasserstoff- oder Methyl-Rest darstellen.

4. Verbindung nach Anspruch 2, worin R¹ einen Wasserstoff-Rest oder einen Alkyl-Rest mit 1-4 Kohlenstoffatomen bedeutet;
R² einen Alkyl-, Cycloalkylalkyl- oder Aralkyl-Rest bedeutet;
R³ Alkyl mit 2 bis 5 Kohlenstoffatomen oder einen Heteroaralkyl-Rest bedeutet; und
R⁴ einen tert.-Butyl-Rest bedeutet.

5. Pharmazeutische Zusammensetzung, die eine Verbindung nach einem der Ansprüche 1 bis 4 und einen pharmazeutisch akzeptablen Träger aufweist.

6. Verwendung einer Verbindung nach einem der Ansprüche 1 bis 4 zur Herstellung eines Medikaments zur Virus-Inhibierung.

7. Verwendung nach Anspruch 6 einer Verbindung, wie in einem der Ansprüche 1 bis 4 definiert, zur Herstellung eines Medikaments zur Inhibierung von retroviraler Protease.

8. Verwendung nach Anspruch 7, wobei die retrovirale Protease HIV-Protease ist.

9. Verwendung nach Anspruch 6 einer Verbindung, wie in einem der Ansprüche 1 bis 4 definiert, zur Herstellung eines Medikaments zur Behandlung einer retroviralen Infektion.

10. Verwendung nach Anspruch 9, wobei die retrovirale Infektion eine HIV-Infektion ist.

11. Verwendung nach Anspruch 6 einer Verbindung, wie in einem der Ansprüche 1 bis 4 definiert, zur Herstellung eines Medikaments zur Behandlung von AIDS.

## Revendications

1. Composé représenté par la formule (I) ou un sel pharmaceutiquement acceptable de ce composé : 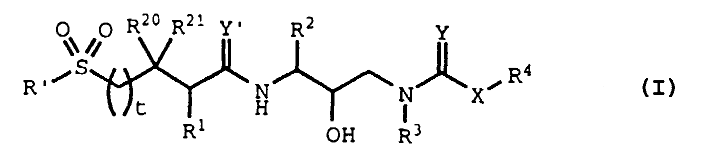 formule dans laquelle :
t représente 0 ou 1 ;
R' représente des radicaux alkyle, aryle ou aralkyle ;
R¹ représente -CH₂SO₂NH₂, un groupe alkyle renfermant de 1 à 4 atomes de carbone ou des radicaux cyclo-alkyle ou la chaîne latérale de l'acide aminé asparagine, la S-méthylcystéine ou les dérivés sulfoxyde ou sulfone de cette dernière, la glycine, l'allo-isoleucine, l'alanine, la leucine, la tert-leucine, la phénylalanine, l'ornithine, la thréonine, l'allo-thréonine, l'isoleucine, l'histidine, la norleucine, la valine, la glutamine, la sérine, l'acide aspartique ou la béta-cyano-alanine ;
R² représente les radicaux alkyle, aryle, cycloalkyle, cycloalkylalkyle ou aralkyle, pouvant être substitués par halogène, par des radicaux -OR⁹ ou SR⁹ dans lesquels R⁹ représente un atome d'hydrogène ou des radicaux alkyle ;
R³ et R⁴ représentent, indépendamment l'un de l'autre, les radicaux alkyle, cycloalkyle, cycloalkylalkyle, hétérocycloalkyle, hétérocycloalkylalkyle, aryle, aralkyle ou hétéroaralkyle ;
R²⁰ et R²¹, indépendamment l'un de l'autre, représentent un atome d'hydrogène ou des radicaux alkyle ;
X représente O et CH(R¹⁷) où R¹⁷ représente un atome d'hydrogène ou des radicaux alkyle ; et
Y et Y' représentent, indépendamment l'un de l'autre, O ou S ; et
où alkyle, seul ou en combinaison, représente un radical alkyle à chaîne droite ou ramifiée contenant de 1 à 8 atomes de carbone ; cycloalkyle, seul ou en combinaison, représente un radical alkyle cyclique contenant de 3 à 8 atomes de carbone, aryle, seul ou en combinaison, représente un radical phényle ou naphtyle non substitué ou bien un radical phényle ou naphtyle substitué par un groupe alkyle en C₁-C₈, un groupe alcoxy en C₁-C₈, de l'halogène, un radical hydroxy ou amino ; hétérocycloalkyle, seul ou en combinaison, représente les radicaux pyrrolidinyle, pipéridinyle, pipérazinyle, morpholinyle, thiamorpholinyle, tétrahydroquinolinyle ou tétrahydroisoquinolinyle, ces radicaux pouvant éventuellement être substitués sur un atome de carbone substituable, par halogène, alkyle en C₁-C₈, alcoxy en C₁-C₈ ou oxo, sur un atome d'azote secondaire d'un noyau, par alkyle en C₁-C₈, (aryle en C₁-C₈)-alcoxycarbonyle, alcanoyle en C₁-C₈, phényle ou phényl-(alkyle en C₁-C₈) ou sur un atome d'azote tertiaire d'un noyau par oxydo ; hétéroaryle représente, seul ou en combinaison, les radicaux pyrrolyle, imidazolyle, pyrazolyle, pyridyle, pyrazinyle, pyrimidinyle, furyle, thiényle, triazolyle, oxazolyle, thiazolyle, indolyle, quinolinyle, isoquinolinyle, quinoxa1inyle, β-carbolinyle, benzofuranyle ou benzimidazolyle, ces radicaux pouvant être éventuellement substitués sur un atome de carbone substituable, par halogène, alkyle en C₁-C₈, alcoxy en C₁-C₈ ou oxo ; sur un atome d'azote secondaire d'un noyau, par alkyle en C₁-C₈, (aryle en C₁-C₈)-alcoxycarbonyle, alcanoyle en C₁-C₈, phényle ou phényl-(alkyle en C₁-C₈), ou sur un atome d'azote tertiaire d'un noyau par oxydo.

2. Composé selon la revendication 1, dans la formule duquel :
t représente 0 ;
R¹ représente un atome d'hydrogène ou un radical alkyle renfermant de 1 à 4 atomes de carbone ; et
R² représente les radicaux alkyle, cycloalkylalkyle ou aralkyle, pouvant éventuellement être substitués par halogène ou par des radicaux -OR⁹ ou SR⁹ où R⁹ représente des radicaux alkyle.

3. Composé selon la revendication 2, dans la formule duquel :
R' représente les radicaux méthyle ou phénéthyle ;
R¹ représente les radicaux méthyle, éthyle, isopropyle ou tert-butyle ;
R² représente les radicaux CH₃SCH₂CH₂-, isobutyle, n-butyle, benzyle, 4-fluorobenzyle, 2-naphtylméthyle ou cyclohexylméthyle ;
R³ représente les radicaux i-propyle, i-butyle, néo-pentyle, i-amyle, t-butyle, n-butyle, cyclohexylméthyle, benzyle, parafluorobenzyle, paraméthoxybenzyle, paraméthylbenzyle et 2-naphtylméthyle ;
R⁴ représente le radical t-butyle ; et
R²⁰ et R²¹, indépendamment l'un de l'autre, représentent un atome d'hydrogène ou un radical méthyle.

4. Composé selon la revendication 2, dans la formule duquel :
R¹ représente un atome d'hydrogène ou un radical alkyle renfermant de 1 à 4 atomes de carbone ; et
R² représente les radicaux alkyle, cycloalkylalkyle ou aralkyle ;
R³ représente un radical alkyle renfermant de 2 à 5 atomes de carbone ou un radical hétéroalkyle ; et
R⁴ représente le radical tert-butyle.

5. Composition pharmaceutique comprenant un composé selon l'une des revendications 1 à 4, ainsi qu'un véhicule pharmaceutiquement acceptable.

6. Utilisation d'un composé selon l'une quelconque des revendications 1 à 4, pour la préparation d'un médicament inhibant les virus.

7. Utilisation selon la revendication 6, d'un composé défini dans l'une quelconque des revendications 1 à 4 pour la préparation de médicaments inhibant la protéase rétrovirale.

8. Utilisation selon la revendications 7, lorsque la protéase rétrovirale est la protéase de VIH.

9. Utilisation selon la revendication 6, d'un composé défini dans l'une quelconque des revendications 1 à 4, pour la préparation de médicaments pour le traitement d'une infection rétrovirale.

10. Utilisation selon la revendication 9 lorsque l'infection rétrovirale est une infection par VIH.

11. Utilisation selon la revendication 6 d'un composé défini dans l'une quelconque des revendications 1 à 4 pour la fabrication de médicaments pour le traitement du SIDA.
